# EUROPEAN PATENT SPECIFICATION

(11) **EP 0 482 607 B1**
(45) Date of publication and mention of the grant of the patent: **04.09.1996**
(21) Application number: 91118067.7
(22) Date of filing: 23.10.1991
(51) Int. Cl.: C07D 277/40

(54) **Process of producing 2-aminothiazole**
Verfahren zur Herstellung von 2-Aminothiazole
Procédé de préparation de 2-amino thiazole

(30) Priority: 25.10.1990 JP 287582/90
(43) Date of publication of application: 29.04.1992
(73) Proprietor: KUREHA CHEMICAL INDUSTRY CO., LTD., Chuo-ku Tokyo 103 (JP)
(72) Inventor: Wakasugi, Takashi, Iwaki-shi, Fukushima-ken (JP); Miyakawa, Tadashi, Iwaki-shi, Fukushima-ken (JP); Tonouchi, Naka, Iwaki-shi, Fukushima-ken (JP); Yamauchi, Takashi, Iwaki-shi, Fukushima-ken (JP)
(74) Representative: Boeters, Hans Dietrich, Dr.

(56) References cited:
- EP-A- 0 368 613
- CH-A- 283 422
- CHEMICAL ABSTRACTS, vol. 51, no. 21, 10 November 1957, Columbus, Ohio, US; abstract no. 16560H, column 16560; & SU-A-106 446

## Description

### FIELD OF THE INVENTION

The present invention relates to a process of producing 2-aminothiazole.

### BACKGROUND OF THE INVENTION

2-Aminothiazole is a compound useful for being used as a synthetic raw material of medicaments, dyes, etc.

Hitherto, 2-aminothiazole is produced by reacting a compound such as, for example, α, β-dichloroethyl acetate, α , β-dichloroethyl ether, etc., capable of easily forming monochloroacetaldehyde (hereinafter, is referred to as MCA) by a hydrolysis and thiourea in an aqueous medium to form 2-aminothiazole and separating the product by crystallization.

However, the compound of forming MCA which is a raw material in the process, such as α , β-dichloroethyl acetate, α , β-dichloroethyl ether, etc., is expensive and also it is difficult to remove the reaction side products formed at reacting the foregoing compound and thiourea. Also, since MCA is an unstable compound, MCA is usually stored as an aqueous solution of from 40 to 45% thereof and in the case of using MCA as a starting material, the reaction is carried out in water or a water-containing medium.

However, since a commercially available aqueous MCA solution contains a large amount of impurities, 2-aminothiazole crystals obtained by reacting and crystallizing in water or a water-containing medium contains several % impurities which are difficult to be separated. Thus, when the 2-aminothiazole crystals are stored as they are, the crystals tend to be decomposed with the passage of time by the influence of the impurities.

Furthermore, since 2-aminothiazole is a relatively unstable compound, the purification thereof is difficult and when it is purified by distillation, the yield thereof is low.

As an example of carrying out the synthesis of 2-aminothiazole using an organic solvent, a method of synthesizing a thiazole-azo compound by carrying out a diazotation reaction in succession to the synthesis of 2-aminothiazole is known as described, e.g., in USP 40467. In the method, 2-aminothiazole is formed by suspending thiourea in glacial acetic acid and adding dropwise an aqueous MCA solution to the suspension to cause the reaction. In this case, however, the reaction mixture at the formation of 2-aminothiazole is an acetic acid solution of 2-aminothiazole containing at least ten and several percent water and 2-aminothiazole is not separated from the acetic acid solution. It is not easy to separate 2-aminothiazole from the acetic acid solution containing more than 10% water and in this case, the yield for 2-aminothiazole in the purification step is also low.

The reason that the conventional synthesis of 2-aminothiazole is carried out in an aqueous medium is that the isolation and storage of high-pure MCA at a high concentration are difficult and also it is very difficult to transfer MCA in an aqueous solution into an organic layer.

To sum up, since 2-aminothiazole obtained by a conventional method is easily soluble in water, the synthesis of 2-aminothiazole in the presence of water makes a purification thereof difficult, and low-purity crystals thereof are obtained only at a low yield.

CA, 51 (1957) 16560 h discloses a process for preparing 2-aminothiazole by reacting crystals of a chloroacetaldehyde dimer and solid thiourea.

An object of this invention is to provide a process of producing 2-aminothiazole, which can be stably stored for a long period of time, at a high purity and at a high yield.

The problem underlying the invention is solved by a process of producing 2-aminothiazole, which comprises reacting monochloroacetaldehyde obtained by depolymerizing a monochloroacetaldehyde trimer and thiourea in an organic solvent having a boiling point of 50 to 120 °C and containing not more than 5 % water.

The invention results from the discovery that high-pure 2-aminothiazole can be synthesized at a high yield by, after once forming a trimer of MCA, which can be stably stored at a high purity, depolymerizing the trimer and reacting MCA thus obtained and thiourea in an organic solvent.

In other words, this invention relates to a process of producing 2-aminothiazole by reacting MCA obtained by depolymerizing a MCA trimer and thiourea in an organic solvent.

In this invention, a MCA trimer is used as the raw material. Since high-pure MCA can be obtained at a high yield by using a MCA trimer, the subsequent thiazoleforming reaction can be carried out in an organic solvent.

### DETAILED DESCRIPTION OF THE INVENTION

Then, the invention is described in detail.

The MCA trimer itself which is the starting material in the process of the present invention is a known material as described in Monatsh., 3, 461-464(1882) and several production processes thereof are known. In a preferred production process, the MCA trimer is easily obtained by dissolving a chlorinated liquid of acetaldehyde containing MCA as the main component in an organic solvent such as hexane and cyclizing three moles of MCA it in the existence of sulfuric acid (USP 5008462).

The MCA trimer thus obtained is a stable compound, which can be stored for a long period of time as it is, and hence in the synthesis reaction system using the MCA trimer as the raw material, an aqueous medium is not required.

As an organic solvent being used in this invention, there is no particular restriction thereon if the solvent is reluctant to react with MCA and thiourea but on considering the subsequent treatment, an organic solvent having a boiling point as low as possible is preferred.

Examples of the organic solvent are alcohols such as methanol, ethanol, propanol, etc.; aliphatic hydrocarbons such as hexane, heptane, etc.; alicyclic hydrocarbons such as cysclohexane, etc.; aromatic hydrocarbons such as benzene, toluene, etc.; carbon tetrachloride and dioxane. These solvents are used in the state that a water content is preferably not more than 3%. If the content of water in the reaction mixture at the end of the synthesis reaction of 2-aminothiazole is more than 10%, the subsequent separation and purification of 2-aminothiazole become difficult, whereby high-pure 2-aminothiazole is not obtained.

At the practice of the process of this invention, a MCA trimer is first depolymerized to form a MCA monomer. For example, when a MCA trimer is heated to a temperature of from 120°C to 130°C in the existence of an acid catalyst such as p-toluenesulfonic acid, high-pure MCA is reproduced. The synthesis of 2-aminothiazole is carried out by placing MCA thus obtained, thiourea and the foregoing organic solvent in a reaction vessel and reacting by heating. The components may be charged in the reaction vessel in any desired order. For example, the organic solvent and thiourea are previously placed in the reaction vessel and after raising the temperature thereto to a reaction temperature, MCA or an organic solution of MCA may be added dropwise to the mixture but it is preferred that after placing definite amounts of MCA, thiourea, and the organic solvent in the reaction vessel, the temperature of the system is raised to carry out the reaction. since the reaction is an equimolar reaction of MCA and thiourea, the charging amount of each reaction component may be almost an equimolar amount. However, usually MCA is used in a slightly excessive amount. The organic solvent may be used from 300 to 800 ml in the reaction of about 1 mol of MCA. The reaction is carried out at a temperature of from 40 °C to a refluxing temperature, and preferably from 45°C to 70°C for from 1 to 3 hours.

After the reaction is over, a neutralizing agent such as sodium hydrogencarbonate, etc., is added to the reaction mixture in an amount of slightly larger than the equivalent amount of bi-produced hydrogen chloride followed by stirring to neutralize the reaction product and after separating sodium chloride and excessive sodium hydrogencarbonate thus precipitated by filtration, the solvent is removed from the reaction mixture, thereby crystals of 2-aminothiazole are deposited. When the crystals are washed with water or an organic solvent such as hexane, etc., or recrystallized from benzene, 2-aminothiazole having a purity of at least 99% can be obtained at a yield of at least 85%(based on thiourea charged).

In addition, when the reaction solvent contains more than ten and several percent water, the filtering property of crystals deposited after removing the solvent is poor since sodium chloride and sodium hydrogencarbonate are dissolved in water, and also for removing these inorganic salts contained in the crystals, washing with water or an extraction operation with an organic solvent is required, which results in the reduction of the production yield.

In the process of this invention, MCA which is essentially unstable is once converted to a stable trimer thereof and at use, MCA obtained by depolymerizing the MCA trimer is used as the raw material, whereby it becomes possible to synthesize 2-aminothiazole in a state of not containing water more than the amount of water formed.

As the result thereof, 2-aminothiazole having a high purity of at least 99% can be industrially advantageously produced. Also, 2-aminothiazole thus obtained can be stored for a long period of time without being decomposed or denatured.

Then, the invention is explained more practically by the following examples and comparison examples but the invention is not limited to these examples.

In addition, the measurement of the purity in the following examples was carried out by an internal standard gas chromatographic method (internal standard material: dibutyl ether).

### Example 1

In a 50 ml three neck distillation flask equipped with a stirrer was placed 25.0 g of a MCA trimer and the trimer was distilled at 130°C in the existence of p-toluenesulfonic acid to distill MCA. As the result of analyzing the composition of 23.85 g of the distillate thus obtained by gas chromatography, it was confirmed that the product was MCA having a purity of 99.9%.

The synthesis of 2-aminothiazole was carried out by placing 22.0 g (0.28 mol) of foregoing MCA, 20.6 g (0.27 mol) of thiourea and 200 ml of 2-propanol (water content: not more than 0.3%) as a solvent in a 300 ml three neck distillation flask equipped with a thermometer, a stirrer, and a reflux condenser and carrying out the reaction for 2.0 hours at 60°C. After the reaction was over, sodium hydrogencarbonate, 25 g (0.30 mol) was gradually added to the reaction mixture and a neutralization was carried out by stirring the resultant mixture at 60°C for 3 hours. After the neutralization, sodium chloride formed and excessive sodium hydrogencarbonate were removed by filtration, the solvent was removed from the solution obtained under reduced pressure to deposite crystals.

The crystals obtained were washed with a smoll amount of water, further washed repeatedly with hexane, and then dried to provide 26.2 g (0.26 mol) of 2-aminothiazole having a purity of 99.9%.

The synthesis yield thereof was 96.3% to thiourea used.

Also, when the 2-aminothiazole obtained was stored in a bath kept at a constant temperature of 40°C for 30 days and the change of the composition was determined by gas chromatography, the purity thereof 99.9% and the change of the composition with the passage of time was not observed.

### Comparison Example 1

In a 300 ml three neck distillation flask equipped with a thermometer, a stirrer and a reflux condenser were placed 55 g of a commercially available aqueous 40% MCA solution (MCA 0.28 mol, made by Tokyo Kasei Kogyo K.K.) and 100 g of an aqueous solution containing 20.6 g (0.27 mol) of thiourea and the reaction was carried out for 5 hours at 60°C.

After the reaction was over, 100 g of an aqueous solution of 20% sodium hydroxide was added to the reaction mixture and after stirring the mixture for 1.0 hour at 30°C, the product formed was extracted 5 times with 200 ml of diethyl ether. The extract thus obtained was dried by anhydrous magnesium sulfate and then the solvent was distilled off to deposit crystals.

The crystals obtained were washed with hexane and then dried to provide 15.8 g (0.15 mol) of 2-aminothiazole having a purity of 96.5%.

The synthesis yield thereof was 55.6% to thiourea used.

Also, when the 2-aminothiazole was stored in a bath kept at a constant temperature of 40°C for 30 days and then the change of the composition was determined by gas chromatography, the purity was found to be lowered to 93.2%.

### Comparison Example 2

In a 300 ml three neck distillation flask equipped with a thermometer, a stirrer and a reflux condenser were placed 55 g of a commercially available aqueous 40% MCA solution (MCA 0.28 mol, made by Tokyo Kasei Kogyo K.K.), 20.6 g (0.27 mol) of thiourea and 200 ml of 2-propanol as a solvent and the reaction was carried out for 5.0 hours at 60 °C.

After the reaction was over, 25 g (0.40 mol) of sodium hydrogencarbonate was gradually added to the reaction mixture and the resultant mixture was stirred for 3.0 hours at 60°C to neutralize the mixture. After neutralization, sodium chloride formed and excessive sodium hydrogencarbonate were removed by filtration and water and solvent were distilled off from the filtrate obtained under reduced pressure to deposit crystals.

The crystals obtained contained the salt formed and water. The crystals were washed with a small amount of water, further washed repeatedly with hexane, and then dried to provide 14.7 g (0.14 mol) of 2-aminothiazole having a purity of 95.8%.

The synthesis yield was 51.9% to thiourea used.

### Example 2

In a 300 mol three neck distillation flask equipped with a thermometer, a stirrer and a reflux condenser were placed 22.0 g (0.28 mol) obtained from a MCA trimer as in Example 1 and 20.6 g (0.27 mol) of thiourea and after adding thereto 200 ml of methanol (water content was not more than 0.07%), the reaction was carried out for 3 hours at 50°C.

After the reaction was over, methanol was distilled off from the reaction mixture to provide 2-aminothiazole hydrochloride. To 2-aminothiazole hydrochloride was added 100 g of an aqueous 20% sodium hydroxide solution and after stirring the mixture for 1.0 hour at 30°C, the reaction product was extracted 5 times with 100 ml of diethyl ether. The extract obtained was dried with anhydrous magnesium sulfate and then the solvent was distilled off from the extract to deposit crystals.

The crystals obtained were recrystallized from benzene to provide 24.3 g (0.24 mol) of 2-aminothiazole having a purity of 99.9%.

The synthesis yield was 88.9% to thiourea used.

### Example 3

In a 300 ml of three neck distillation flask equipped with a thermometer, a stirrer and a reflux condenser were placed 22.0 g (0.28 mol) of MCA obtained from a MCA trimer as in Example 1 and 20.6 g (0.27 mol) of thiourea and after adding thereto 200 ml of benzene (water content was not more than 0.04%) as a solvent, the reaction was carried out for 3 hours at 50°C.

After the reaction was over, 100 g of an aqueous 20% sodium hydroxide was added to the reaction mixture and the mixture was shaked. After separating a benzene layer formed, the benzene layer was concentrated and allowed to stand to provide 23.2 g (0.23 mol) of 2-aminothiazole having a purity of 99.9%.

The synthesis yield was 85.2% to thiourea used.

### Example 4

The same procedure as Example 1 was followed except that 200 ml of methanol (water content was not more than 0.07%) was used as the solvent.

The purity of 2-aminothiazole obtained was 99.8% and the synthesis yield thereof was 92.6% to thiourea used.

## Claims

1. A process of producing 2-aminothiazole, which comprises reacting monochloroacetaldehyde obtained by depolymerizing a monochloroacetaldehyde trimer and thiourea in an organic solvent having a boiling point of 50 to 120 °C and containing not more than 5 % water.

2. The process of producing 2-aminothiazole as claimed in claim 1, wherein the organic solvent is selected from the group consisting of alcohols, aliphatic hydrocarbons, alicyclic hydrocarbons, aromatic hydrocarbons, carbon tetrachloride and dioxane.

3. The process of producing 2-aminothiazole as claimed in claim 1 or 2, wherein the depolymerization of the monochloroacetaldehyde trimer is carried out by heating it to a temperature of from 120 to 130 °C in the presence of an acid catalyst.

4. The processs of producing 2-aminothiazole as claimed in any of the preceding claims, wherein the reaction of monochloroacetaldehyde and thiourea in an organic solvent is carried out for of from 1 to 3 hours at a temperature of from 40 °C up to the refluxing temperature.

5. The process of producing 2-aminothiazole as claimed in claim 4, wherein after the reaction, the reaction mixture is neutralized, precipitates formed are removed by filtration, and the solvent is distilled off from the filtrate.

## Patentansprüche

1. Verfahren zur Herstellung von 2-Aminothiazol, bei dem man Monochloracetaldehyd, erhalten durch Depolymerisieren eines Monochloracetaldehydtrimeren, und Thioharnstoff in einem organischen Lösungsmittel mit einem Siedepunkt von 50 bis 120 °C und mit einem Gehalt von nicht mehr als 5 % Wasser umsetzt.

2. Verfahren zur Herstellung von 2-Aminothiazol nach Anspruch 1, bei dem man das organische Lösungsmittel aus der durch Alkohole, aliphatische Kohlenwasserstoffe, alicyclische Kohlenwasserstoffe, aromatische Kohlenwasserstoffe, Tetrachlorkohlenstoff und Dioxan gebildeten Gruppe wählt.

3. Verfahren zur Herstellung von 2-Aminothiazol nach Anspruch 1 oder 2, bei dem man die Depolymerisation des Monochloracetaldehydtrimeren dadurch durchführt, daß man es auf eine Temperatur von 120 bis 130 °C in Gegenwart eines Säurekatalysators erhitzt.

4. Verfahren zur Herstellung von 2-Aminothiazol nach einem der vorhergehenden Ansprüche, bei dem man die Umsetzung von Monochloracetaldehyd und Thioharnstoff in einem organischen Lösungsmittel 1 bis 3 h lang bei einer Temperatur von 40 °C bis zur Rückflußtemperatur durchführt.

5. Verfahren zur Herstellung von 2-Aminothiazol nach Anspruch 4, bei dem man nach der Umsetzung die Reaktionsmischung neutralisiert, gebildete Niederschläge durch Filtration entfernt und das Lösungsmittel vom Filtrat abdestilliert.

## Revendications

1. Procédé de préparation de 2-aminothiazole, qui comporte le fait de faire réagir du monochloroacétaldéhyde, obtenu par dépolymérisation de trimère de monochloroacétaldéhyde, et de la thiourée, dans un solvant organique dont le point d'ébullition vaut de 50°C à 120°C et qui ne contient pas plus de 5 % d'eau.

2. Procédé de préparation de 2-aminothiazole, conforme à la revendication 1, dans lequel le solvant organique est choisi parmi les alcools, les hydrocarbures aliphatiques, les hydrocarbures alicycliques, les hydrocarbures aromatiques, le tétrachlorure de carbone et le dioxane.

3. Procédé de préparation de 2-aminothiazole, conforme à la revendication 1 ou 2, dans lequel on réalise la dépolymérisation du trimère de monochloroacétaldéhyde en le chauffant à une température de 120°C à 130°C, en présence d'un catalyseur acide.

4. Procédé de préparation de 2-aminothiazole, conforme à l'une des revendications précédentes, dans lequel on fait réagir, dans un solvant organique, le monochloroacétaldéhyde et la thiourée pendant 1 à 3 heures et à une température située entre 40°C et la température de reflux.

5. Procédé de préparation de 2-aminothiazole, conforme à la revendication 4, dans lequel, une fois la réaction terminée, on rend neutre le mélange réactionnel, on élimine par filtration le précipité formé et l'on chasse le solvant du filtrat par distillation.
